(19) Europäisches Patentamt
European Patent Office
Office européen des brevets

(11) Numéro de publication: **0 179 679**
**B1**

(12) **FASCICULE DE BREVET EUROPÉEN**

(45) Date de publication du fascicule du brevet: **29.03.89**

(21) Numéro de dépôt: **85401740.7**

(22) Date de dépôt: **06.09.85**

(51) Int. Cl.⁴: **C 12 P 19/04,** C 12 P 21/00, A 61 K 31/715

(54) **Polysaccharides membranaires utiles notamment comme médicament et procédé pour leur préparation.**

(30) Priorité: **10.09.84 FR 8413844**

(43) Date de publication de la demande: **30.04.86 Bulletin 86/18**

(45) Mention de la délivrance du brevet: **29.03.89 Bulletin 89/13**

(84) Etats contractants désignés: **AT BE CH DE FR GB IT LI LU NL SE**

(56) Documents cité:
**EP-A-0 049 182**
**EP-A-0 089 266**

**CHEMICAL ABSTRACTS, vol. 99, no. 23 décembre 1983, page 615, abrégé no. 193207y, Columbus, Ohio, US**

(73) Titulaire: **PIERRE FABRE MEDICAMENT, 125, Rue de la Faisanderie, F-75116 Paris (FR)**

(72) Inventeur: **Dussourd d'Hinterland, Lucien, Domaine de Plombières Avenue de Lautrec, F-81100 Castres (FR)**
Inventeur: **Normier, Gérard, 23, rue Francis Poullenc, F-81100 Castres (FR)**
Inventeur: **Pinel, Anne- Marie, 22, rue des Capucins, F-81100 Castres (FR)**

(74) Mandataire: **Warcoin, Jacques, Cabinet Régimbeau 26, avenue Kléber, F-75116 Paris (FR)**

**EP 0 179 679 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1989

## Description

La présente invention concerne un polysaccharide de faible poids moléculaire (environ 90 000) obtenu à partir de protéoglycanes membranaires d'origine bactérienne ainsi qu'un procédé pour sa préparation et son utilisation à titre de médicament, en particulier col immunostimulant, notamment pour l'activation des cellules NK (Natural Killer), et comme adjuvant dans les vaccins.

Le brevet français n° 7 835 649 déposé le 19 décembre 1978 décrit un protéoglycane membranaire détoxifié, isolé d'une souche mutante non capsulée et non pathogène de Klebsiella pneumoniae biotype a. Ce protéoglycane possède un poids moléculaire supérieur à 2 000 000 de daltons et exerce des propriétés adjuvantes caractéristiques vis-à-vis de la production d'anticorps contre les antigènes qui lui sont associés. Il ne possède par contre pas d'activité stimulante notable des cellules NK. Des exemples sont donnés de son utilisation comme adjuvant dans des formules de vaccins.

Le brevet français n° 8 203 921 déposé le 9 mars 1982 décrit l'obtention, à partir du protéoglycane précédent et par action du lysozyme, d'une fraction protéoglycanique de poids moléculaire compris entre 200 000 et 400 000 daltons. Cette fraction protéoglycanique possède une propriété nouvelle celle de stimuler très fortement l'activité des cellules NK via une induction d'interféron endogène. Cette fraction, par contre, ne possède plus les propriétés adjuvantes du protéoglycane détoxifié décrit précédemment.

La présente invention concerne un nouveau composé provenant, en particulier, de membranes bactériennes; il s'agit d'un polysaccharide ayant un poids moléculaire compris entre 80 000 et 100 000 daltons et ayant la composition suivante sous forme lyophilisée:

| | | |
|---|---|---|
| . teneur en galactose, | 66 ± 6 | % |
| . teneur en hexoses autres que le galactose, | < 1 | % |
| . teneur en hexosamines (glucosamine), | 8,5 ± 2 | % |
| . teneur en amino-acides, | 5 ± 2 | % |
| . teneur en acides gras, | < 1 | % |
| . teneur en acides nucléiques, | < 0,005 | % |
| . teneur en protéines, | < 0,3 | % |

Ce nouveau polysaccharide obtenu à partir de protéoglycanes membranaires d'origine bactérienne solubles est caractérisé par un poids moléculaire faible (90 000 ± 10 000) et par sa composition particulière; il ne contient, en effet, qu'un seul hexose, le galactose, et une seule hexosamine, la glucosamine, sur laquelle sont branchés des peptides correspondant aux acides aminés analysés. Comme en outre ce polysaccharide est pratiquement exempt d'acides gras, d'acides nucléiques et de protéines, il s'agit d'un produit bien caractérisé dont la structure a d'ailleurs pu être déterminée.

L'étude de la structure du polysaccharide au moyen des méthodes classiques telles que oxydation périodique, méthylation, chromatographie gazeuse couplée à la spectrométrie de masse, RMN, etc., a permis de définir de façon précise la séquence d'enchaînement de ses constituants.

Les résultats obtenus montrent que ce polysaccharide est formé de l'enchaînement répété d'unités monomériques ayant la structure suivante:

$$\left[\rightarrow (3\text{-}Gal_f\text{-}(1\rightarrow 3)\text{-}Gal_p\text{-}(1\rightarrow 3)\text{-}Gal_p\text{-}(1\rightarrow 3)\text{-}Gal_p\text{-}(1\rightarrow 3)\text{-}Gal_p\text{-}(1\rightarrow 3)\text{-}Gal_p\text{-}(1\rightarrow 3)\right] Gal_p(1\rightarrow$$

$$\begin{array}{c} 4 \\ \uparrow \\ 1 \\ Gal_p \end{array}$$

où $Gal_f$ = galacto furanose,

$Gal_p$ = galacto pyranose (sous formes α et β),

ce qui correspond à la formule développée suivante:

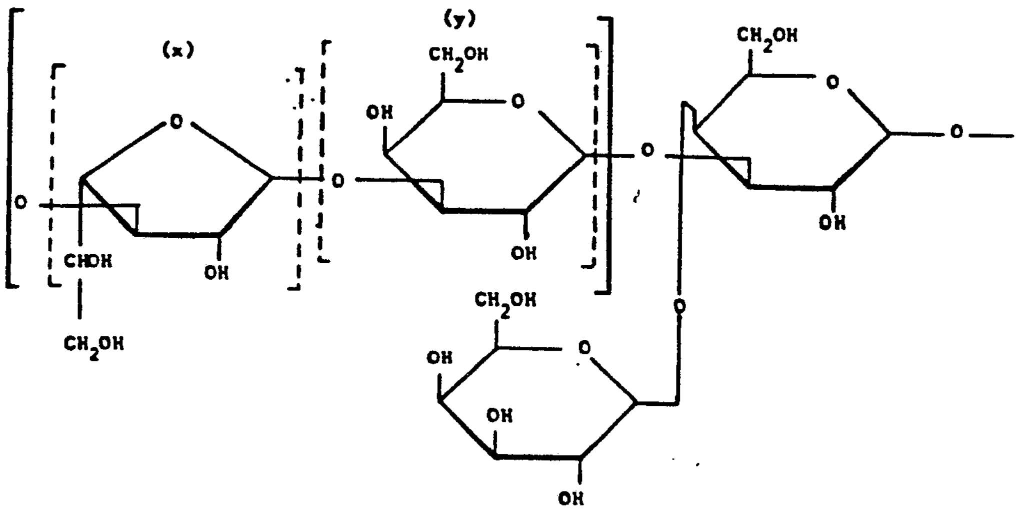

où x 1, y = 6.

Ce polysaccharide peut être préparé par différents procédés.

Le procédé préféré selon la présente invention, qui permet de préparele polysaccharide membranaire d'origine bactérienne tel que décrit précédement est caracterisé en ce que:

a) à partir d'une souche de bactérie gram négatif, on extrait des membranes les protéoglycanes solubles dans l'eau;

b) à partir desdits protéoglycanes solubles, on isole des polysaccharides de poids moléculaire compris entre 80 000 et 100 000; et

c) on purifie, si nécessaire, les polysaccharides isolés en éliminant les protéines présentes dans la fraction isolée.

Parmi les bactéries gram négatif susceptibles d'être mises en oeuvre, on peut citer Klebsiella pneumoniae, Serratia marcescens et Escherichia coli; en particulier Klebsiella pneumoniae qui fait l'objet d'un dépot à la Collection Nationale de Culture de Microorganismes (CNCM) sous le n° 145-I-IP.

Les protéoglycanes solubles sont préparés, de préférence, par solubilisation de protéoglycanes membranaires bruts obtenus par la mise en oeuvre de procédés décrits dans la technique antérieure, en particulier par les procédés décrits dans le brevet français n° 7 835 649. Il s'agit, essentiellement, d'un procédé qui, à partir d'un broyat cellulaire, sépare les membranes par plusieurs centrifugations.

Les protéoglycanes bruts obtenus sont traités par une base, en particulier un hydroxyde alcalin, de préférence la soude, à une molarité comprise entre 0,3 et 1 M, de préférence 0,5 M, le traitement étant poursuivi quelques heures, par exemple 1 heure, à une température comprise entre 50 et 60° C, par exemple 56° C, de préférence sous agitation.

Par rapport au procédé décrit dans le brevet français n° 7 835 649, le milieu utilisé est plus basique pour assurer une hydrolyse plus complète des protéoglycanes bruts.

Afin d'éliminer l'excès de réactif, après refroidissement, on neutralise la suspension par un acide, par exemple l'acide chlorhydrique, puis on la clarifie en éliminant le résidu insoluble par précipitation, en particulier par centrifugation, par exemple 60 minutes à 30 000 g.

Le surnageant, qui constitue les protéoglycanes solubles, est recueilli et peut être lyophilisé.

Le polysaccharide membranaire d'origine bactérienne avant le poids moléculaire désiré est alors isolé du surnageant par un procédé de fractionnement, de préférence un fractionnement chromatographique. On recueille la fraction d'élution de la colonne contenant le polysaccharide de poids moléculaire compris entre 80 000 et 100 000.

Ce fractionnement chromatographique peut être effectué en utilisant une résine d'agarose telle que la Sépharose CL2B (marque commerciale Pharmacia, Suède), mais d'autres techniques de fractionnement peuvent être mises en oeuvre.

Le polysaccharide peut, alors, être purifié, dans un mode de réalisation de l'invention, en éliminant en particulier les protéines de poids moléculaire proche de celui du polysaccharide isolé, par exemple par hydrolyse enzymatique desdites protéines, en particulier par action de protéinase, suivie d'une séparation des polysaccharides purifiés par un procédé de fractionnement, de préférence un fractionnement chromatographique.

La fraction contenant le polysaccharide peut être alors recueillie et éventuellement lyophilisée. Le lyophilisat ainsi obtenu constitue le polysaccharide faisant l'objet de la présente invention.

La présente invention concerne également l'utilisation de ces protéoglycanes à titre de médicament, soit seuls, soit en association avec un agent vaccinant.

En effet, le polysaccharide selon l'invention possède des propriétés immunostimulantes dont les principes sont:

. un pouvoir adjuvant, notamment à des doses très faibles, des vaccins, en particulier ribosomaux;

. une capacité élevée de stimulation des cellules NK, in vitro et in vivo.

Ainsi, il a été mis en évidence pour ces produits une forte activation des cellules NK in vivo sur des souris vis-à-vis du lymphome de Moloney (cellule YAC.1).

En outre, on a mis en évidence des propriétés adjuvantes vis-à-vis des vaccins, notamment des vaccins ribosomaux, c'est-à-dire les vaccins dans lesquels l'agent vaccinant est constitué par des ribosomes, mais d'autres agents vaccinants peuvent être utilisés.

Il est clair, compte tenu des activités mises en évidence, que la nature des compositions pharmaceutiques ainsi que les posologies à mettre en oeuvre, pourront varier dans une très large gamme. En effet, l'activation des cellules NK pourra dépendre grandement des cellules cibles visées et de l'état du patient. De même, dans le cas des adjuvants, seule une étude d'optimisation pourra permettre de fixer le rapport entre l'adjuvant et l'agent vaccinant ainsi que les posologies.

Les exemples ci-après permettront de mettre en évidence d'autres caractéristiques et avantages de la présente invention.

Les figures ci-annexées illustrent certains aspects particuliers de l'invention. Sur ces figures:

. la figure 1 est une courbe chromatographique sur CL2B Sépharose du protéoglycane hydrolysé par NaOH 0,5 N,

. la 2 est une courbe chromatographique sur Séphacryl S 200 (marque commerciale) polysaccharide traité par la protéinase,

. la figure 3 représente les courbes d'activation des cellules NK in vitro (exemple 6),

. la figure 4 représente les courbes d'activation des cellules NK in vivo (exemple 7),

. la figure 5 représente un histogramme du dosage ELISA obtenu dans l'exemple 8.

**Exemple 1**

Isolement des protéoglycanes membrainaires bruts

La biomasse de la souche de Klebsiella pneumoniae 145-I-IP est dispersée dans du tampon Tris HCl 0,01 M, pH 7, contenant NaCl 0,15 M glacé puis soumise à un broyage mécanique destiné à rompre les parois cellulaires.

Le lysat bactérien est clarifié par une centrifugation de 10 minutes à 7 500 g puis le surnageant est centrifugé pendant 60 minutes à 30 000 g.

Le culot est dispersé dans une solution aqueuse de NaCl 0,15 M. La suspension obtenue est à nouveau clarifiée 10 minutes à 7 500 g puis centrifugée 45 minutes à 30 000 g.

Le culot est repris dans l'eau distillée puis à nouveau soumis à un cycle de centrifugation à 7 500 g puis à 30 000 g.

Le culot de protéoglycanes membranaires bruts est alors repris dans 1/4 du volume initial d'eau distillée stérile, la suspension est clarifiée 10 minutes à 7 500 g et le surnageant est lyophilisé.

**Exemple 2**

Préparation de protéoglycanes membranaires solubles

Les protéoglycanes membranaires bruts lyophilisés sont dispersés dans NaOH (0,5 M) puis hydrolysés pendant 1 heure à 56°C. Après refroidissement, la suspension est neutralisée par HCl. La suspension est alors clarifiée par centrifugation 60 minutes à 30 000 g puis le surnageant est recueilli. Le filtrat est lyophilisé.

**Exemple 3**

Isolement du polysaccharide par fractionnement chromatographique

Le lyophilisat précèdent est dissous dans du tampon Tris HCl 0,01 M, pH 7, puis soumis à un premier fractionnement chromatographique sur une colonne (60 x 2,6 cm) de Sépharose CL2B (marque commerciale Pharmacia, Suède) (gel d'agarose de porosité déterminée) équilibrée dans le même tampon.

La figure 1 donne un exemple de la séparation chromatographique obtenue à ce stade avec indication de la localisation précise du pic de polysaccharide représenté en pointillé.

On remarque sur ce chromatogramme que le pic de polysaccharide est complètement séparé d'un pic de poids moléculaire très supérieur élué en tête de la colonne et incomplètement séparé d'un ensemble de

fractions protéiques d'un poids moléculaire inférieur proche de celui du polysaccharide, éluées à la suite de ce dernier.

La fraction d'élution de la colonne contenant le polysaccharide est recueillie, dialysée contre de l'eau distillée puis lyophilisée.

Cette fraction est encore légèrement contaminée par des protéines de poids moléculaire très proche de celui du polysaccharide (70 000 à 100 000). Ces protéines vont donc être hydrolysées par l'action d'une enzyme avant une ultime étape de fractionnement chromatographique.

**Exemple 4**

Hydrolyse enzymatique des protéines résiduelles

Le lyophilisat précèdent est dissous dans du tampon Tris HCl 0,01 M, pH 7,3, contenant 1 mM d'EDTA à raison de 20 mg/ml. On ajoute alors à cette solution 50 mg/ml de protéinase K (enzyme protéolytique de Tritirachium album) puis on incube pendant 2 heures à 37° C sous agitation.

**Exemple 5**

Purification des polysaccharides par fractionnement chromatographique

Les protéines contaminantes dont le poids moléculaire a été réduit par protéolyse sont séparées du polysaccharide par chromatographie sur Séphacryl S 200 (marque commerciale Pharmacia, Suède) (gel de polyacrylamide de porosité définie).

La figure 2 donne un exemple de la séparation chromatographique obtenue à ce stade.

On remarque sur ce chromatogramme que le pic de polysaccharide représenté en pointillé se trouve cette fois complètement séparé de celui des protéines hydrolysées représentées en trait plein, dont l'élution de la colonne se trouve ainsi fortement retardée par rapport au polysaccharide.

La fraction contenant le polysaccharide pur est alors recueillie, concentrée et dialysée en continu contre de l'eau distillée sur une membrane dont le seuil de coupure se situe a 10 000 daltons, stérilisée par filtration sur membrane 0,2 μ puis lyophilisée.

Le lyophilisat ainsi obtenu constitue le polysaccharide de Klebsiella pneumoniae faisant l'objet du présent brevet.

**Exemple 6**

Activation des cellules NK in vitro

Protocole

$10^7$ cellules spléniques des souris normales par ml de milieu RPMI 1640 additionné de 5 % de sérum foetal de veau.

Incubation à 37° C dans une étuve à $CO_2$ avec des quantités variables de polysaccharide.

Les cellules cibles sont des cellules YAC-1 (lymphome de Moloney) sensibles aux cellules NK, marquées au $^{51}Cr$.

La mesure de la lyse est faite par comptage du $^{51}Cr$ libéré pour des rapports cellules effectrices/cellules cibles de 200/1, 100/1, 50/1, pendant 4 heures d'incubation.

Résultats

Ils sont extrêmement nets et font apparaître une augmentation hautement significative du pourcentage de cellules cibles lysées ($P < 0,01$) dès la dose de 1 μg/ml.

La figure 3 donne un exemple des résultats obtenus au cours de cette étude.

**Exemple 7**

Activation des cellules NK in vivo par voie intrapéritonéale chez la souris

Protocole

Animaux: souris CBA/J, âgées de 4 à 5 mois.

Cellules cibles: YAC-1 (lymphome de Moloney) sensibles aux cellules NK.

Le polysaccharide est injecté par voie intrapéritonéale à la dose de 15 μg dans 0,2 ml de sérum physiologique tamponné 3 jours avant la mesure de l'activité NK. Des souris témoins ne reçoivent que du sérum physiologique dans les mêmes conditions.

Les cellules spléniques sont ensuite prélevées et incubées comme précédemment avec des cellules cibles

marquées au $^{51}Cr$ dans des rapports cellules effectrices/cellules cibles de 200/1, 100/1, 50/1, 25/1 pendant 4 heures. Le $^{51}Cr$ libéré est mesuré pour déterminer le pourcentage de cellules cibles lysées.

Résultats

On observe une augmentation hautement significative de l'activité NK chez les animaux traités par le polysaccharide comparativement aux animaux témoins.

La figure 4 donne un exemple des résultats obtenus au cours de cette étude.

**Exemple 8**

Propriétés adjuvantes du polysaccharide

Exemple d'étude des propriétés adjuvantes du polysaccharide vis-à-vis d'un antigène ribosomal de Streptococcus pyogenes groupe A.

Principe

Des souris sont immunisées avec une dose constante de ribosomes de Streptococcus pyogenes additionnés de doses variables de polysaccharide. La réponse anticorps spécifiques contre les ribosomes est ensuite mesurée par la technique ELISA.

Des groupes témoins sont traités l'un par du sérum physiologique, l'autre par la dose standard de protéoglycane membranaire brut de Klebsiella pneumiae (PGM brut).

Protocole

9 lots de 10 souris NMRI femelles âgées de 8 semaines. Chaque animal reçoit 5 injections en 15 jours.

| | | | | |
|---|---|---|---|---|
| . lot 1 : sérum | physiologique | | | |
| . lot 2 : 10 µg | de ribosomes | + 0,05 | µg | de polysaccharide |
| . lot 3 : 10 µg | " | + 0,1 | µg | " |
| . lot 4 : 10 µg | " | + 0,5 | µg | " |
| . lot 5 : 10 µg | " | + 1,0 | µg | " |
| . lot 6 : 10 µg | " | + 2,5 | µg | " |
| . lot 7 : 10 µg | " | + 5,0 | µg | " |
| . lot 8 : 10 µg | " | +10,0 | µg | " |
| . lot 9 : 10 µg | " | +15,0 | µg | de PGM bruts |

Les animaux sont ponctionnés le 21 ème jour et les titres en anticorps anti-ribosomes de Streptococcus pyogènes sont déterminés par la technique ELISA.

Les résultats indiquent la dernière dilution du sérum donnant une réponse positive en ELISA, et sont représentés sous forme d'histogramme ci-après.

Résultats

La figure 5 est une représentation sous forme d'histogramme du dosage ELISA de la réponse anticorps spécifique en fonction de la dose de polysaccharide associée à l'antigène.

Antigène: 10 µg de ribosomes de S. pyogenes par injection.

Ces résultats montrent qu'une dose de 2,5 µg de polysaccharide à le même effet adjuvant qu'une dose de 15 µg de PGM bruts, soit une activité spécifique 6 fois supérieure dans ces conditions.

**Revendications**

1. Polysaccharide membranaire d'origine bactérienne ayant pour composition analytique quantitative:

| | | |
|---|---|---|
| . teneur en galactose, | 66 ± 6 | % |
| . teneur en hexoses autres que le galactose, | < 1 | % |
| . teneur en hexosamines (glucosamine), | 8,5 ± 2 | % |
| . teneur en amino-acides, | 5 ± 2 | % |
| . teneur en acides gras, | < 1 | % |
| . teneur en acides nucléiques, | < 0,005 | % |
| . teneur en protéines, | < 0,3 | % |

et pour poids moléculaire: 90 000 ± 10 000.

2. Polysaccharide selon la revendication 1 ayant la structure polymère définie par le monomère:

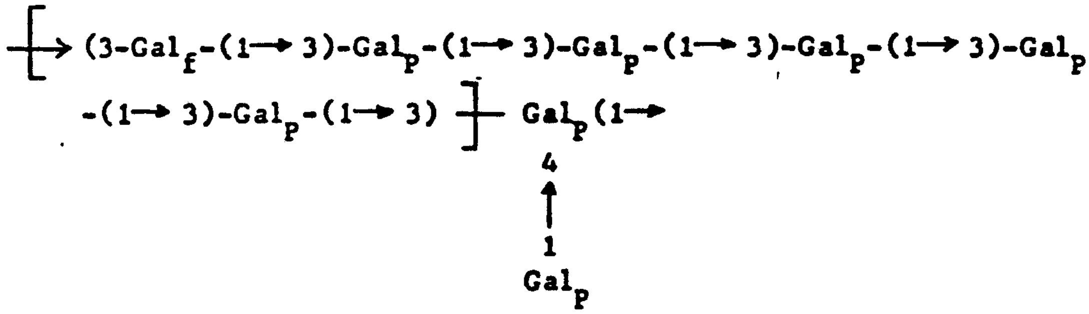

où $Gal_f$ = galacto furanose,
$Gal_p$ = galacto pyranose (sous forme α et β).

3. Polysaccharide selon la revendication 2 où le monomère est représenté par la formule développée:

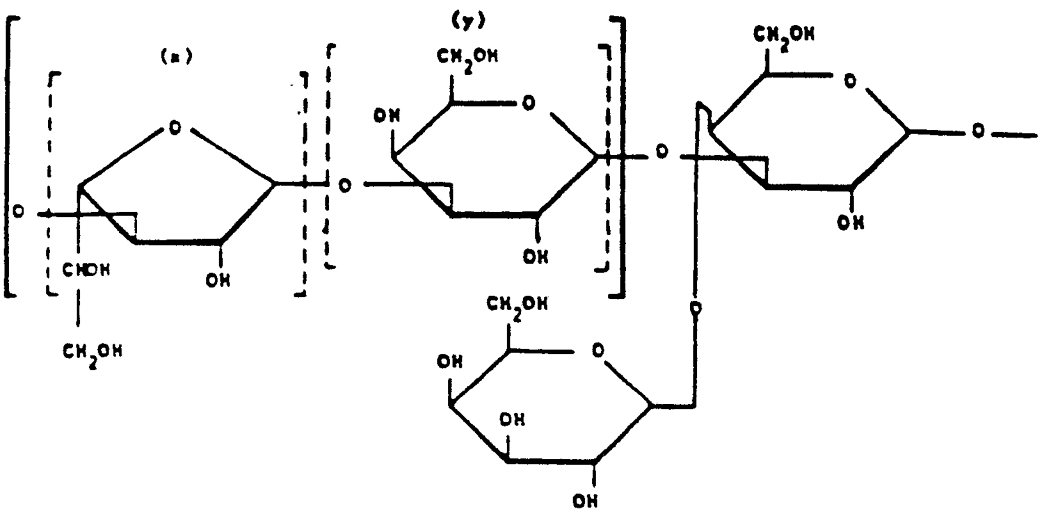

où x = 1 ; y = 6.

4. Procédé de préparation de polysaccharide membranaire d'origine bactérienne selon l'une des revendications 1 à 3 caractérisé en ce que:

a) à partir d'une souche de bactérie gram négatif, on extrait des protéoglycanes membranaires solubles dans l'eau;

b) des protéoglycanes solubles, on isole des polysaccharides de poids moléculaire compris entre 80 000 et 100 000; et

c) on purifie, si nécessaire, les polysaccharides isolés en éliminant les protéines présentes dans la fraction isolée.

5. Procédé selon la revendication 4, caractérisé en ce que la bactérie gram négatif est un Klebsiella pneumoniae.

6. Procédé selon l'une des revendications 4 ou 5, caractérisé en ce que dans l'étape a) d'obtention des protéoglycanes solubles dans l'eau, on traite des protéoglycanes bruts membranaires par une base, on récupère les protéoglycanes solubles qui se trouvent dans la solution aqueuse.

7. Procédé selon l'une des revendications 4 a 6, caractérisé en ce que dans l'étape a) la base utilisée est un hydroxyde alcalin de molarité 0,3 M à 1 M.

8. Procédé selon l'une des revendications 4 a 7, caractérisé en ce que dans l'étape b) on isole les polysaccharides par fractionnement chromatographique en recueillant la fraction d'élution contenant les polysaccharides de poids moléculaire compris entre 80 000 et 100 000.

9. Procédé selon l'une des revendications 4 a 8, caractérisé en ce que dans l'étape c) on élimine les protéines présentes dans la fraction isolée par l'action d'une protéinase, suivie d'un fractionnement chromatographique après lequel on recueille la fraction d'élution contenant les polysaccharides de poids moléculaire compris entre 80 000 et 100 000.

10. Polysaccharide d'origine bactérienne obtenu par le procédé selon l'une quelconque des revendications 4 à 9.

11. Médicament caractérisé en ce qu'il contient un polysaccharide selon l'une des revendications 1, 2, 3 ou 10.

12. Médicament selon la revendication 11, caractérisé en ce qu'il contient, en outre, un agent vaccinant.

13. Composition pharmaceutique caractérisée en ce qu'elle comporte au moins un médicament selon l'une des revendications 11 et 12 ainsi qu'un support pharmaceutiquement acceptable.

## Claims

1. Membrane polysaccharide of bacterial origin having the following quantitative analytical composition:

| | | |
|---|---|---|
| . galactose content | 66 ± 6 | % |
| . content of hexoses other than galactose | < 1 | % |
| . hexosamine (glucosamine) content | 8.5 ± 2 | % |
| . amino acid content | 5 ± 2 | % |
| . fatty acid content | < 1 | % |
| . nucleic acid content | < 0.005 | % |
| . protein content | < 0.3 | % |

and molecular weight: 90,000 ± 10,000.

2. Polysaccharide of polymeric structure as claimed in claim 1 defined by the monomer:

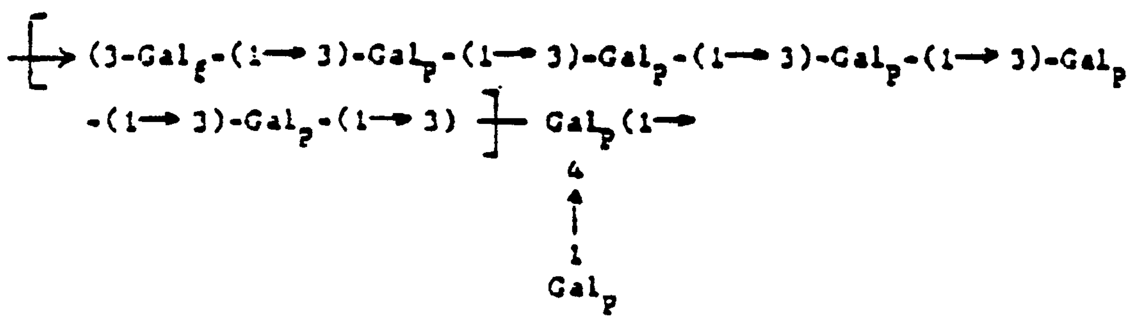

where $Gal_f$ = galactofuranose
$Gal_p$ = galactopyranose (in α and β form).

3. Polysaccharide as claimed in claim 2, in which the monomer is represented by the structural formula:

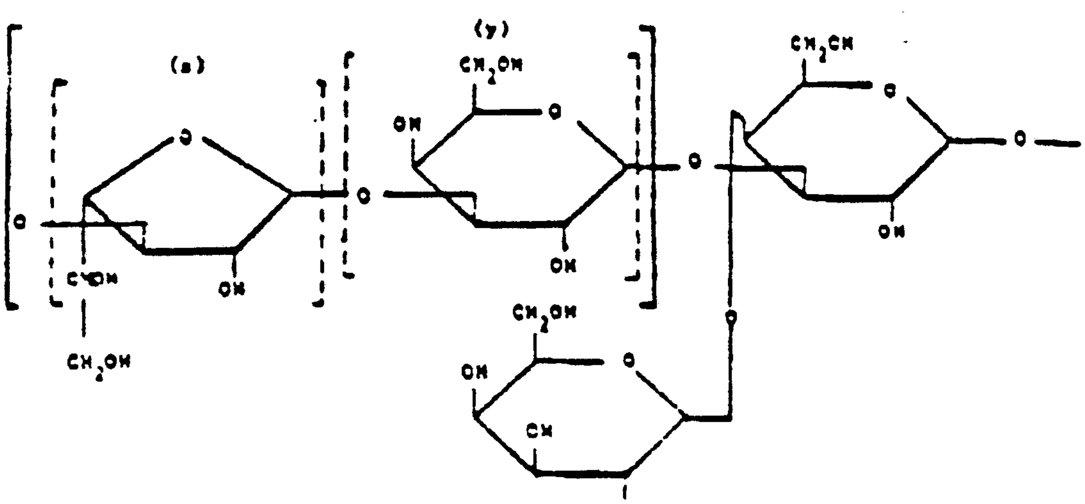

where x = 1; y = 6.

4. Process for preparing a membrane polysaccharide of bacterial origin as claimed in one of claim 1 to 3, wherein:

a) starting wich a strain of a gram-negative batterium, the water-soluble membrane proteoglycans are extracted;

b) from the said soluble proteoglycans, polysaccharides of molecular weight between 80,000 and 100,000 are isolated; and

c) if necessary, the isolated polysaccharides are purified by removing the proteins present in the fraction isolated.

5. Process as claimed in claim 4, wherein the gram-negative bacterium is a Klebsiella pneumoniae.

6. Process as claimed in claims 4 or 5, wherein, in the stage a) for obtaining the water-soluble proteoglycans, crude membrane proteoglycans are treated with a base, and the soluble proteoglycans present in the aqueous solution are recovered.

7. Process as claimed in one of claims 4 to 6, wherein, in stage a), the base used is an alkali metal hydroxide of molarity 0.3 M to 1 M.

8. Process as claimed in one of claims 4 to 7, wherein, in stage b), the polysaccharides are isolated by chromatographic fractionation, collecting the elution fraction containing the polysaccharides of molecular weight between 80,000 and 100,000.

9. Process as claimed in one of claims 4 to 8, wherein, in stage c), the proteins present in the isolated fraction arc removed by the action of a proteinase, followed by a chromatographic fractionation after which the elution fraction containing the polysaccharides of molecular weight between 80,000 and 100,000 is collected.

10. Polysaccharide of bacterial origin obtained by the process as claimed in any one of claims 4 to 9.

11. Drug which contains a polysaccharide as claimed in one of claims 1, 2, 3 or 10.

12. Drug as claimed in claim 11, which contains, in addition, a vaccinating agent.

13. Pharmaceutical composition which contains at least one drug as claimed in one of claims 11 and 12, as well as a pharmaceutically acceptable carrier.

**Patentansprüche**

1. Membranöses Plysaccharid bakteriellen Ursprungs von folgender quantitativen analytischen Zusammensetzung:

| | | |
|---|---|---|
| . Gehalt an Galaktose, | 66 ± 6 | % |
| . Gehalt an von Galaktose verschiedenen Hexosen | < 1 | % |
| . Gehalt an Hexosaminen (Glukosaminen) | 8,5 ± 2 | % |
| . Gehalt an Aminosäuren | 5 ± 2 | % |
| . Gehalt an Fettsäuren | < 1 | % |
| . Gehalt an Nukleinsäuren | < 0,005 | % |
| . Gehalt an Proteinen | < 0,3 | % |

und einem Molekulargewicht von: 90 000 ± 10 000.

2. Polysaccharid nach Anspruch 1 mit einer Polymerstruktur bestimmt durch das Monomer:

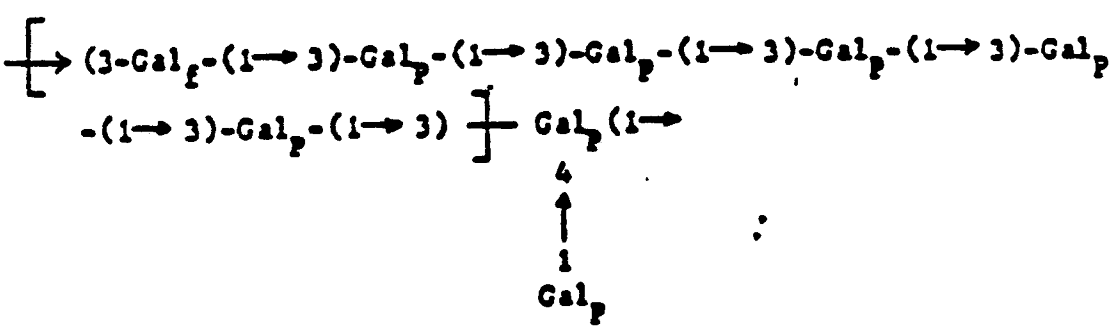

worin bedeutet: $Gal_f$ = Galactofuranose,
$Gal_p$ = Galactopyranose (mit der α- und der β-Form).

3. Polysaccharid nach Anspruch 2, in dem das Monomer der folgenden Strukturformel entspricht:

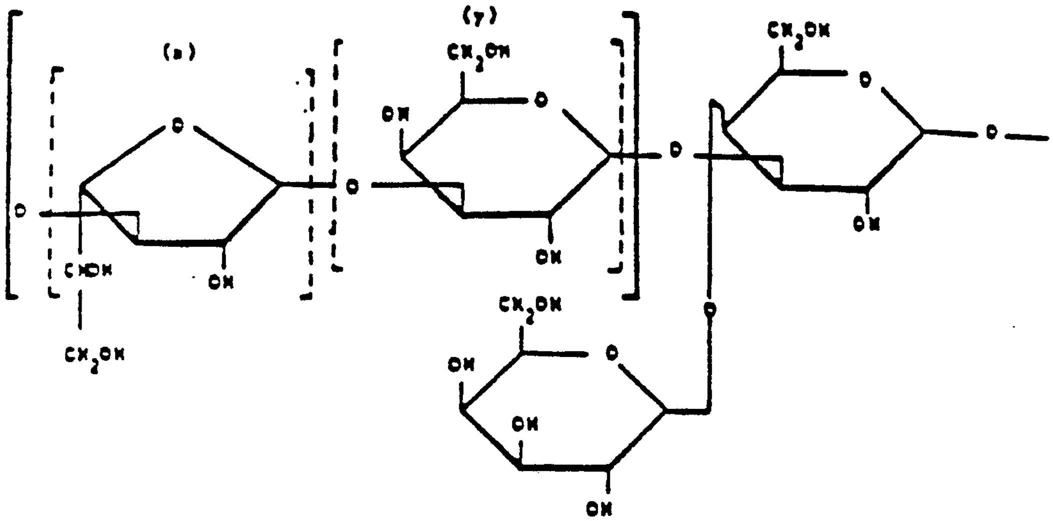

in der x = 1 und y = 6 bedeuten.

4. Verfahren zur Herstellung eines membranösen Polysaccharides bakteriellen Ursprungs nach einem der Ansprüche 1 bis 3, dadurch gekennzeichnet, daß man:

a) ausgehend von einem Stamm eines gramnegativen Bakteriums in Wasser lösliche membranöse Proteoglycane extrahiert;

b) von den löslichen Proteoglycanen Polysaccharide mit einem Molekulargewicht zwischen 80 000 und 100 000 isoliert; und daß man

c) falls erforderlich, die isolierten Polysaccharide reinigt, indem man in der isolierten Fraktion vorhandene Proteine entfernt.

5. Verfahren nach Anspruch 4, dadurch gekennzeichnet, daß man als gramnegatives Bakterium ein Klebsiella pneumoniae Bakterium verwendet.

6. Verfahren nach einem der Ansprüche 4 oder 5, dadurch gekennzeichnet, daß man in der Stufe a) zur Gewinnung der in Wasser löslichen Proteoglycane die rohen membranösen Proteoglycane mit einer Base behandelt und daß man die löslichen Proteoglycane gewinnt, die sich in der wäßrigen Lösung finden.

7. Verfahren nach einem der Ansprüche 4 bis 6, dadurch gekennzeichnet, daß die in der Stufe a) verwendete Base ein basisches Hydroxid mit einer Molarität von 0,3 M bis 1 M ist.

8. Verfahren nach einem der Ansprüche 4 bis 7, dadurch gekennzeichnet, daß man in der Stufe b) die Polysaccharide durch chromatographische Fraktionierung isoliert, indem man die bei der Eluierung anfallende Fraktion aufnimmt, die Polysaccharide mit einem Molekulargewicht zwischen 80 000 und 100 000 enthält.

9. Verfahren nach einem der Ansprüche 4 bis 8, dadurch gekennzeichnet, daß man in der Stufe c) die in der isolierten Fraktion vorhandenen Proteine durch Einwirkung einer Proteinase isoliert, nachfolgend nach einer chromatographischen Fraktionierung, bei welcher man die bei der Eluierung anfallende Fraktion aufnimmt, die die Polysaccharide mit einem Molekulargewicht zwischen 80 000 und 100 000 enthält.

10. Polysaccharid bakteriellen Ursprungs, erhalten nach einem Verfahren gemäß einem der Ansprüche 4 bis 9.

11. Arzneimittel, dadurch gekennzeichnet, daß es ein Polysaccharid nach einem der Ansprüche 1, 2, 3 oder 10 enthält.

12. Arzneimittel nach Anspruch 11, dadurch gekennzeichnet, daß es zusätzlich ein Vaccine-Mittel enthält.

13. Pharmazeutische Zusammensetzung, dadurch gekennzeichnet, daß sie mindestens ein Arzneimittel nach einem der Ansprüche 11 und 12 sowie einen pharmazeutisch verträglichen Träger enthält.

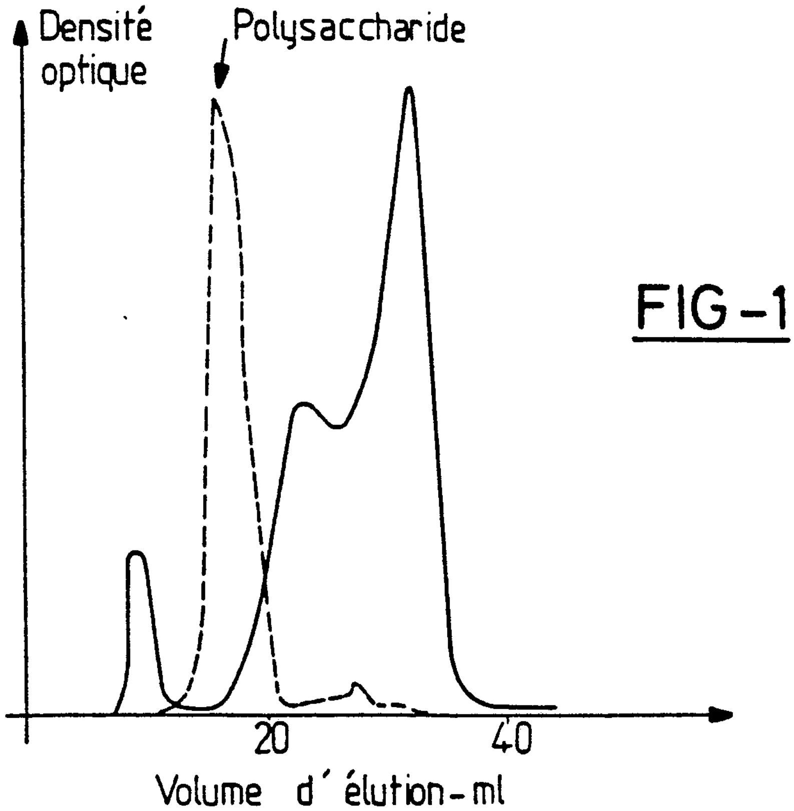
Densité optique
Polysaccharide
20
40
Volume d'élution-ml

FIG-1

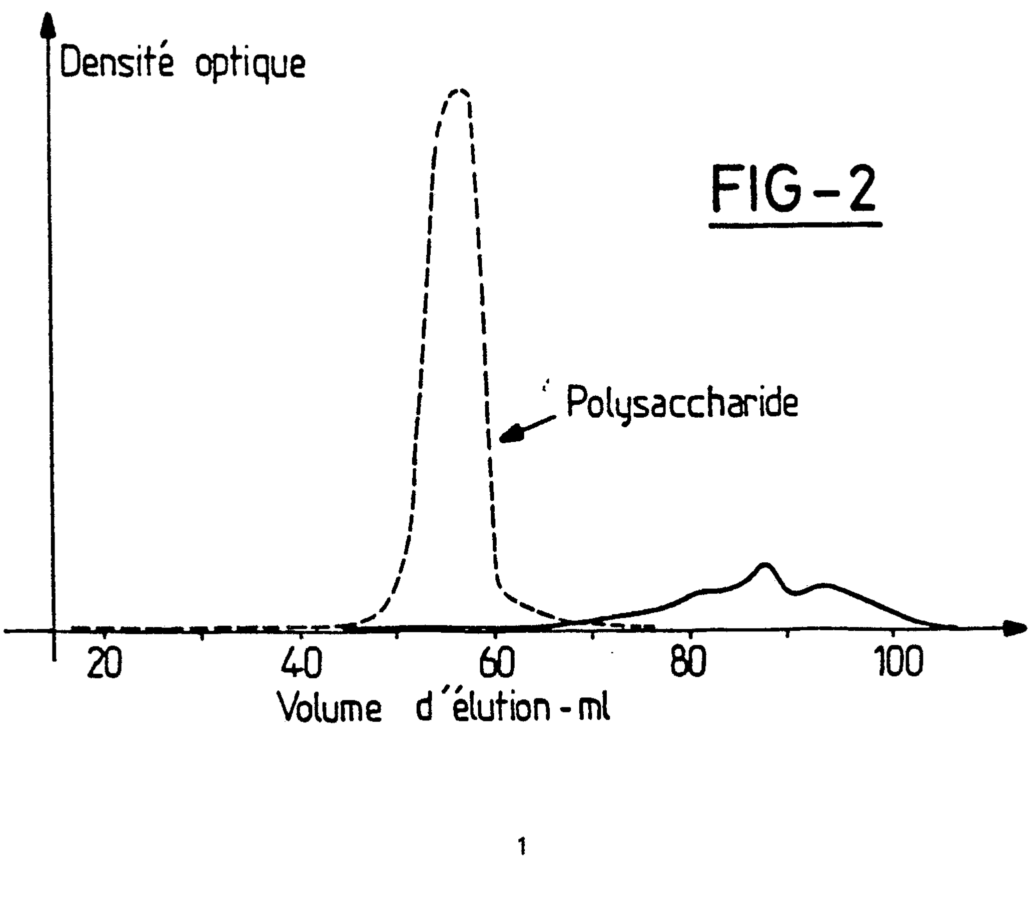
Densité optique
Polysaccharide
20
40
60
80
100
Volume d'élution-ml

FIG-2

FIG-3

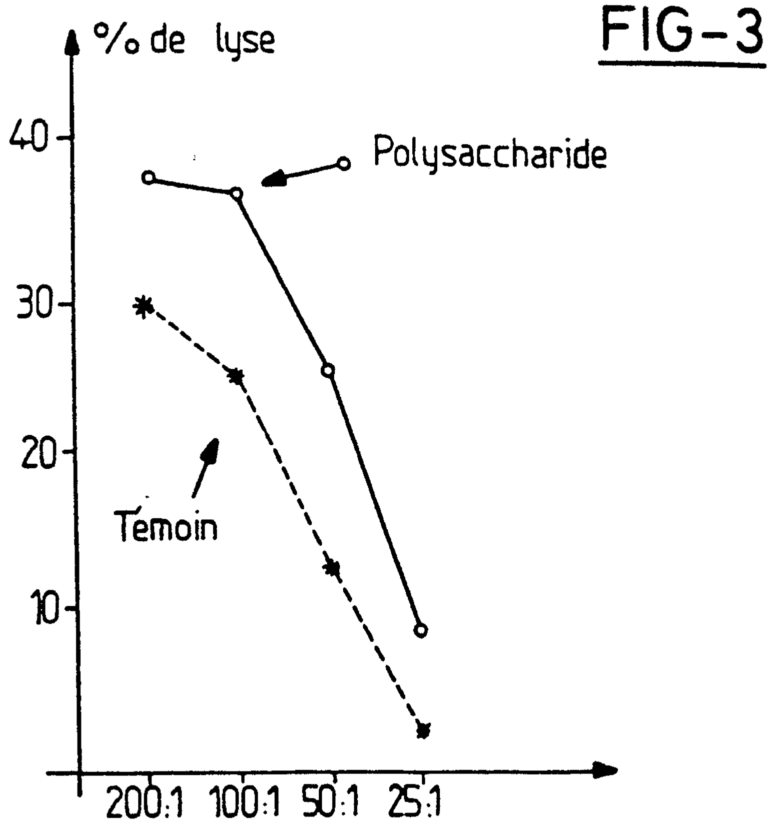
% de lyse
40
30
20
10
Polysaccharide
Témoin
200:1
100:1
50:1
25:1

FIG-4

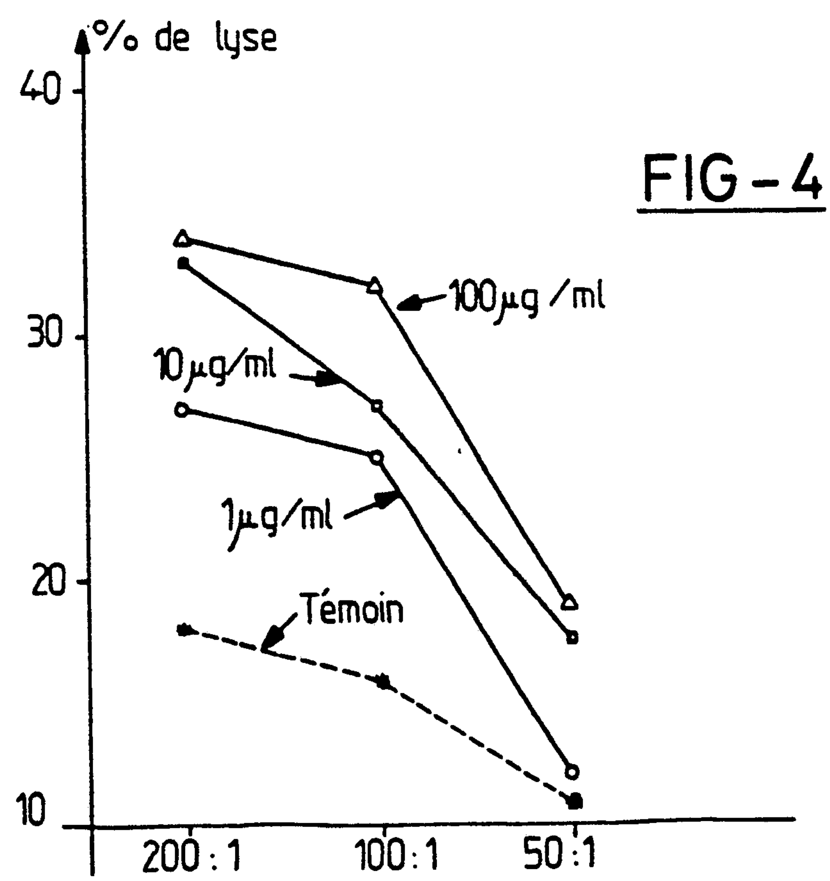
% de lyse
40
30
20
10
100µg /ml
10µg/ml
1µg/ml
Témoin
200 : 1
100 : 1
50 : 1

FIG - 5

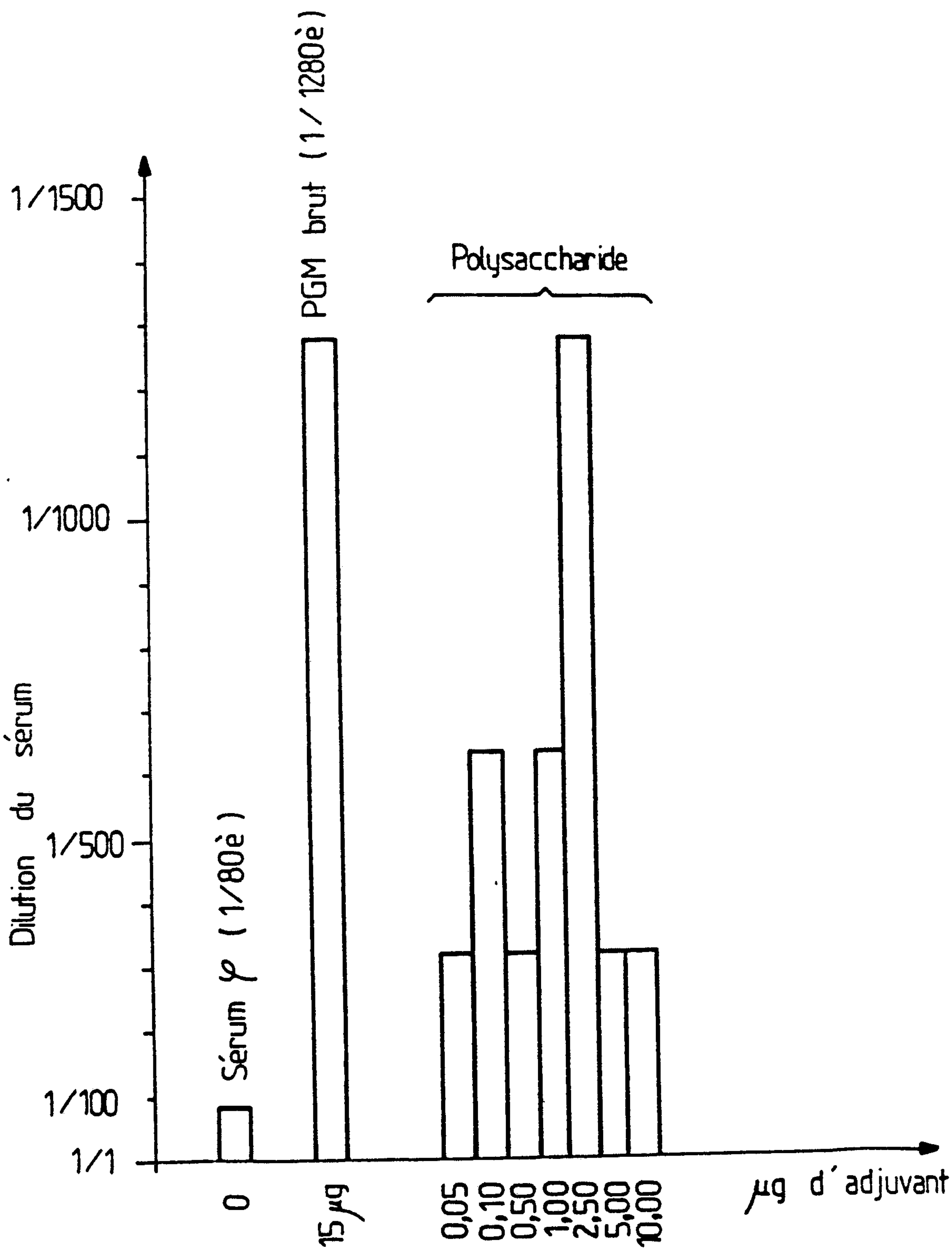
Dilution du sérum
1/1500
1/1000
1/500
1/100
1/1
Sérum ♀ (1/80è)
PGM brut (1/1280è)
Polysaccharide
0
15 µg
0,05
0,10
0,50
1,00
2,50
5,00
10,00
µg d'adjuvant